# EUROPEAN PATENT SPECIFICATION

(11) **EP 0 908 466 B1**
(45) Date of publication and mention of the grant of the patent: **26.04.2006**
(21) Application number: 97922183.5
(22) Date of filing: 26.05.1997
(51) Int. Cl.: C07K 16/30, C12N 5/20, G01N 33/53, G01N 33/577, C12P 21/08, C12N 15/06, C12N 1/21, C12N 5/06, C12P 21/02, A61K 39/395, C12R 1/19, C12R 1/91

(54) **ANTI-HUMAN LECT2 ANTIBODY, CELLS PRODUCING THE SAME, AND METHOD AND KIT FOR ASSAYING THE SAME**
ANTIKÖRPER GEGEN MENSCHLICHES LECT2, ZELLEN DIE DIESEN PRODUZIEREN UND VERFAHREN UND KIT ZU DESSEN BESTIMMUNG
ANTICORPS ANTI-LECT2 HUMAINS, CELLULES LE PRODUISANT ET SES PROCEDE ET MATERIEL DE DOSAGE

(30) Priority: 27.05.1996 JP 13216096
(43) Date of publication of application: 14.04.1999
(73) Proprietor: Medical & Biological Laboratories Co., Ltd., Nagayo-shi, Aichi 460 (JP)
(72) Inventor: ARAI, Takao, Science University of Tokyo, Noda-shi, Chiba 278 (JP)
(74) Representative: Goddar, Heinz J.
(86) International application number: PCT/JP1997/001775
(87) International publication number: WO 1997/045451

(56) References cited:
- JP-A- 8 140 683
- JOURNAL OF INTERFERON RESEARCH, (1993), Vol. 13, YAMAGOE S. et al., "Identification of a Novel Chemotactic Protein for Polymorphonuclear Leukocytes Produced by a Human T Cell Leukemia Cell Line", page S76.
- IMMUNOLOGY LETTERS, (August 1996), Vol. 52, No. 1, YAMAGOE S. et al., "Purification and Primary Amino Acid Sequence of a Novel Neutrophil Chemotactic Factor LECT2", pages 9-13.
- DATABASE BIOSIS PREVIEWS ON DIALOG, BIOSIS, Biosis Number: 98396665, YAMAGOE S. et al., "Novel Homologous Polymorphonuclear Leukocytes Activating Proteins (LECT2) Derived from Human T-Cell Leukemia SKW-3 Cells"; & 9TH INTERNATIONAL CONGRESS OF IMMUNOLOGY, 23-29 July 1995.

### SEQUENCE LISTING

(1) GENERAL INFORMATION:
   (i) APPLICANT:
      (A) NAME: Medical & Biological Laboratories Co., LTD.
      (B) STREET: Sumitomoshojimarunouchi Bldg., 5F., 5-10,
         Nishiki 3-chome
      (C) CITY: Naka-ku, Nagoya-shi
      (D) STATE: Aichi
      (E) COUNTRY: Japan
      (F) POSTAL CODE (ZIP): 460
   (ii) TITLE OF INVENTION: Anti-Human Lect2 Antibody, Cells Producing the Same, and Method and Kit for Assaying the Same
   (iii) NUMBER OF SEQUENCES: 9
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: JP 8-132160
      (B) FILING DATE: 27-MAY-1996
(2) INFORMATION FOR SEQ ID NO: 1:
   (i) SEQUENCE CHARACTERISTICS:
      (A) LENGTH: 151 amino acids
      (B) TYPE: amino acid
      (C) STRANDEDNESS: single
      (D) TOPOLOGY: linear
   (ii) MOLECULE TYPE: protein
   (ix) FEATURE
      (D) OTHER INFORMATION: Xaa = Val or Ile
   (xi) SEQUENCE DESCRIPTION: SEQ ID NO: 1:
   Met Phe Ser Thr Lys Ala Leu Leu Leu Ala Gly Leu Ile Ser Thr Ala
   1               5                   10                  15
   Leu Ala Gly Pro Trp Ala Asn Ile Cys Ala Gly Lys Ser Ser Asn Glu
               20                  25                  30
   Ile Arg Thr Cys Asp Arg His Gly Cys Gly Gln Tyr Ser Ala Gln Arg
           35                  40                  45
   Ser Gln Arg Pro His Gln Gly Val Asp Xaa Leu Cys Ser Ala Gly Ser
       50                  55                  60
   Thr Val Tyr Ala Pro Phe Thr Gly Met Ile Val Gly Gln Glu Lys Pro
   65                  70                  75                  80
   Lys Gln Asn Lys Asn Ala Ile Asn Asn Gly Val Arg Ile Ser Gly Arg
                   85                  90                  95
   Gly Phe Cys Val Lys Met Phe Tyr Ile Lys Pro Ile Lys Tyr Lys Gly
               100                 105                 110
   Pro Ile Lys Lys Gly Glu Lys Leu Gly Thr Leu Leu Pro Leu Gln Lys
           115                 120                 125
   Val Tyr Pro Gly Ile Gln Ser His Val His Ile Glu Asn Cys Asp Ser
       130                 135                 140
   Ser Asp Pro Thr Ala Tyr Leu
   145                 150
(2) INFORMATION FOR SEQ ID NO: 2:
   (i) SEQUENCE CHARACTERISTICS:
      (A) LENGTH: 1092 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: human
      (G) CELL TYPE: liver
   (ix) FEATURE:
      (A) NAME/KEY: 5'UTR
      (B) LOCATION: 1..200
      (C) IDENTIFICATION METHOD: P
   (ix) FEATURE:
      (A) NAME/KEY: 3'UTR
      (B) LOCATION:657..1092
      (C) IDENTIFICATION METHOD: P
   (ix) FEATURE:
      (A) NAME/KEY: CDS
      (B) LOCATION:201..656
      (C) IDEBNTIFICATION METHOD: P
   (ix) FEATURE:
      (A) NAME/KEY: mutation
      (B) LOCATION:replace(372, "a")
      (C) IDENTIFICATION MEHTOD: E
   (ix) FEATURE:
      (A) NAME/KEY: mutation
      (B) LOCATION:replace(748, "g")
      (C) IDENTIFICATION METHOD: E
   (ix) FEATURE:
      (A) NAME/KEY: mutation
      (B) LOCATION:replace(961, "c")
      (C) IDENTIFICATION METHOD: E
   (ix) FEATURE:
      (A) NAME/KEY: mutation
      (B) LOCATION:replace(967, "c")
      (C) IDENTIFICATION METHOD: E
   (ix) FEATURE:
      (A) NAME/KEY: polyA_signal
      (B) LOCATION:684..689
      (C) IDENTIFICATION METHOD: E
   (ix) FEATURE:
      (A) NAME/KEY: polyA_signal
      (B) LOCATION:1060..1065
      (C) IDENTIFICATION METHOD: E
   (xi) SEQUENCE DESCRIPTION: SEQ ID NO: 2:
   AAATCAAATA GCTATCCATG GAATATTAGA ACTTGACTTG CTCCATCCTC TTAAACTTTT     60
   TGTGTCTCAC ACTAAAGAAA TGAGAGATGC AGAATTCTAA GGCTAAATAG CTAGGAAGTA    120
   TTCATTCAAA CTTGAATATC TTCAAAGAGA GTGTGGGGGC AACTCTAATC AGAGGAAGAA    180
   ACTAAAGGAA GTAAAACCAG ATG TTT TCC ACC AAA GCC CTC CTT TTG GCT GGT    233
                         Met Phe Ser Thr Lys Ala Leu Leu Leu Ala Gly
                           1               5                  10
   CTG ATT TCT ACC GCA CTG GCA GGG CCA TGG GCT AAT ATA TGT GCT GGC      281
   Leu Ile Ser Thr Ala Leu Ala Gly Pro Trp Ala Asn Ile Cys Ala Gly
               15                  20                  25
   AAG TCT TCC AAT GAG ATC CGG ACG TGT GAC CGC CAT GGC TGT GGA CAG      329
   Lys Ser Ser Asn Glu Ile Arg Thr Cys Asp Arg His Gly Cys Gly Gln
           30                  35                  40
   TAC TCT GCT CAA AGA AGT CAG AGG CCT CAC CAG GGT GTG GAC GTC TTG      377
   Tyr Ser Ala Gln Arg Ser Gln Arg Pro His Gln Gly Val Asp Val Leu
       45                  50                  55
   TGC TCT GCT GGA TCT ACT GTG TAC GCA CCA TTC ACT GGA ATG ATT GTG      425
   Cys Ser Ala Gly Ser Thr Val Tyr Ala Pro Phe Thr Gly Met Ile Val
   60                  65                  70                  75
   GGC CAG GAG AAA CCT TAT CAA AAC AAG AAT GCT ATC AAT AAT GGT GTT      473
   Gly Gln Glu Lys Pro Tyr Gln Asn Lys Asn Ala Ile Asn Asn Gly Val
                   80                  85                  90
   CGA ATA TCT GGA AGA GGT TTT TGT GTC AAA ATG TTC TAC ATT AAG CCA      521
   Arg Ile Ser Gly Arg Gly Phe Cys Val Lys Met Phe Tyr Ile Lys Pro
               95                 100                 105
   ATT AAG TAT AAA GGT CCT ATT AAG AAG GGA GAA AAA CTT GGA ACT CTA      569
   Ile Lys Tyr Lys Gly Pro Ile Lys Lys Gly Glu Lys Leu Gly Thr Leu
           110                 115                 120
   TTG CCC TTG CAG AAA GTT TAT CCT GGC ATA CAA TCG CAT GTG CAC ATT      617
   Leu Pro Leu Gln Lys Val Tyr Pro Gly Ile Gln Ser His Val His Ile
       125                 130                 135
   GAA AAC TGT GAC TCG AGT GAC CCT ACT GCA TAC CTG TAAATCGAAG           663
   Glu Asn Cys Asp Ser Ser Asp Pro Thr Ala Tyr Leu
   140                 145                 150
   GCCAATGGTC AGATCTTCAA AATAAAAAGT CATCTTAAAA ACCTGGATGC ATACCCTTCT    723
   CTTCAAGAAA TTTGTGTTCA CAAAAGAAAA ATGCATGAAG GGATGGATAC CCCATTTTCC    783
   ATGACATGAT TATTACACAT TGCATGCCTG TATCAAAACA TCTCACGTAC CTCATAAACA    843
   TATACACCTA TGTACCCACA AAAATTTTTT AATTAAAAAA AGGAAATTTG AGTTTAAATA    903
   GAAACATGAT AAATGCAAGA AAGAAAACAT TTTGATTTTA ACTCATTGTC ACTCTGATGT    963
   TCATGTGAAC TGGTTGCTTC GGGCTCTTTG ATCTGTCACC TATGGAATCT GAGTGGTTTT   1023
   ATTTTTTAGA TTTCTCAGTC CCAAAGATCT AAGATAAATA AACAAGAGAA CTTAAAAAAA   1083
   AAAAAAAAA                                                          1092
(2) INFORMATION FOR SEQ ID NO: 3:
   (i) SEQUENCE CHARACTERISTICS:
      (A) LENGTH: 1092 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: human
      (G) CELL TYPE: liver
   (ix) FEATURE:
      (A) NAME/KEY: 5'UTR
      (B) LOCATION: 1..200
      (C) IDENTIFICATION METHOD: P
   (ix) FEATURE:
      (A) NAME/KEY: 3'UTR
      (B) LOCATION:657..1092
      (C) IDENTIFICATION METHOD: P
   (ix) FEATURE:
      (A) NAME/KEY: CDS
      (B) LOCATION:201..656
      (C) IDEBNTIFICATION METHOD: P
   (ix) FEATURE:
      (A) NAME/KEY: mutation
      (B) LOCATION:replace(372, "a")
      (C) IDENTIFICATION MEHTOD: E
   (ix) FEATURE:
      (A) NAME/KEY: mutation
      (B) LOCATION:replace(748, "g")
      (C) IDENTIFICATION METHOD: E
   (ix) FEATURE:
      (A) NAME/KEY: mutation
      (B) LOCATION:replace(961, "c")
      (C) IDENTIFICATION METHOD: E
   (ix) FEATURE:
      (A) NAME/KEY: mutation
      (B) LOCATION:replace(967, "c")
      (C) IDENTIFICATION METHOD: E
   (ix) FEATURE:
      (A) NAME/KEY: polyA_signal
      (B) LOCATION:684..689
      (C) IDENTIFICATION METHOD: E
   (ix) FEATURE:
      (A) NAME/KEY: polyA_signal
      (B) LOCATION: 1060..1065
      (C) IDENTIFICATION METHOD: E
   (xi) SEQUENCE DESCRIPTION: SEQ ID NO: 3:
   AAATCAAATA GCTATCCATG GAATATTAGA ACTTGACTTG CTCCATCCTC TTAAACTTTT    60
   TGTGTCTCAC ACTAAAGAAA TGAGAGATGC AGAATTCTAA GGCTAAATAG CTAGGAAGTA   120
   TTCATTCAAA CTTGAATATC TTCAAAGAGA GTGTGGGGGC AACTCTAATC AGAGGAAGAA   180
   ACTAAAGGAA GTAAAACCAG    ATG TTT TCC ACC AAA GCC CTC CTT TTG GCT GGT 233
                            Met Phe Ser Thr Lys Ala Leu Leu Leu Ala Gly
                              1               5                  10
   CTG ATT TCT ACC GCA CTG GCA GGG CCA TGG GCT AAT ATA TGT GCT GGC     281
   Leu Ile Ser Thr Ala Leu Ala Gly Pro Trp Ala Asn Ile Cys Ala Gly
                   15                  20                  25
   AAG TCT TCC AAT GAG ATC CGG ACG TGT GAC CGC CAT GGC TGT GGA CAG     329
   Lys Ser Ser Asn Glu Ile Arg Thr Cys Asp Arg His Gly Cys Gly Gln
               30                  35                  40
   TAC TCT GCT CAA AGA AGT CAG AGG CCT CAC CAG GGT GTG GAC ATC TTG     377
   Tyr Ser Ala Gln Arg Ser Gln Arg Pro His Gln Gly Val Asp Ile Leu
       45                  50                  55
   TGC TCT GCT GGA TCT ACT GTG TAC GCA CCA TTC ACT GGA ATG ATT GTG     425
   Cys Ser Ala Gly Ser Thr Val Tyr Ala Pro Phe Thr Gly Met Ile Val
   60                  65                  70                  75
   GGC CAG GAG AAA CCT TAT CAA AAC AAG AAT GCT ATC AAT AAT GGT GTT     473
   Gly Gln Glu Lys Pro Tyr Gln Asn Lys Asn Ala Ile Asn Asn Gly Val
                   80                  85                  90
   CGA ATA TCT GGA AGA GGT TTT TGT GTC AAA ATG TTC TAC ATT AAG CCA     521
   Arg Ile Ser Gly Arg Gly Phe Cys Val Lys Met Phe Tyr Ile Lys Pro
                   95                 100                 105
   ATT AAG TAT AAA GGT CCT ATT AAG AAG GGA GAA AAA CTT GGA ACT CTA     569
   Ile Lys Tyr Lys Gly Pro Ile Lys Lys Gly Glu Lys Leu Gly Thr Leu
               110                 115                 120
   TTG CCC TTG CAG AAA GTT TAT CCT GGC ATA CAA TCG CAT GTG CAC ATT     617
   Leu Pro Leu Gln Lys Val Tyr Pro Gly Ile Gln Ser His Val His Ile
       125                 130                 135
   GAA AAC TGT GAC TCG AGT GAC CCT ACT GCA TAC CTG TAAATCGAAG          663
   Glu Asn Cys Asp Ser Ser Asp Pro Thr Ala Tyr Leu
   140                 145                 150
   GCCAATGGTC AGATCTTCAA AATAAAAAGT CATCTTAAAA ACCTGGATGC ATACCCTTCT   723
   CTTCAAGAAA TTTGTGTTCA CAAAAGAAAA ATGCATGAAG GGATGGATAC CCCATTTTCC   783
   ATGACATGAT TATTACACAT TGCATGCCTG TATCAAAACA TCTCACGTAC CTCATAAACA   843
   TATACACCTA TGTACCCACA AAAATTTTTT AATTAAAAAA AGGAAATTTG AGTTTAAATA   903
   GAAACATGAT AAATGCAAGA AAGAAAACAT TTTGATTTTA ACTCATTGTC ACTCTGATGT   963
   TCATGTGAAC TGGTTGCTTC GGGCTCTTTG ATCTGTCACC TATGGAATCT GAGTGGTTTT  1023
   ATTTTTTAGA TTTCTCAGTC CCAAAGATCT AAGATAAATA AACAAGAGAA CTTAAAAAAA  1083
   AAAAAAAAA                                                          1092
(2) INFORMATION FOR SEQ ID NO: 4:
   (i) SEQUENCE CHARACTERISTICS:
      (A) LENGTH: 41 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthesized DNA (cDNA probe)"
   (ix) FEATURE:
      (A) NAME/KEY: misc_RNA
      (B) LOCATION: 6
      (D) OTHER INFORMATION:/ "N = C or G
   (ix) FEATURE:
      (A) NAME/KEY: misc_RNA
      (B) LOCATION:9
      (D) OTHER INFORMATION:/note= "N = A or G"
   (ix) FEATURE:
      (A) NAME/KEY: misc_RNA
      (B) LOCATION:15
      (D) OTHER INFORMATION:/note= "N = T or C"
   (ix) FEATURE:
      (A) NAME/KEY: misc_RNA
      (B) LOCATION:21
      (D) OTHER INFORMATION:/note= "N = C or G"
   (ix) FEATURE:
      (A) NAME/KEY: misc_RNA
      (B) LOCATION:24
      (D) OTHER INFORMATION:/note= "N = T or C"
   (ix) FEATURE:
      (A) NAME/KEY: misc_RNA
      (B) LOCATION:27
      (D) OTHER INFORMATION:/note= "N = T or A"
   (ix) FEATURE:
      (A) NAME/KEY: misc_RNA
      (B) LOCATION:30
      (D) OTHER INFORMATION:/note= "N = C or G"
   (ix) FEATURE:
      (A) NAME/KEY: misc_RNA
      (B) LOCATION:36
      (D) OTHER INFORMATION:/note= "N = T or C"
   (ix) FEATURE:
      (A) NAME/KEY: misc_RNA
      (B) LOCATION:39
      (D) OTHER INFORMATION:/note= "N = T or C"
   (xi) SEQUENCE DESCRIPTION: SEQ ID NO: 4:
   GATGTNCTNT GCTCNGATGG NTCNACNGTN TATGCNCCNT T 41
(2) INFORMATION FOR SEQ ID NO: 5:
   (i) SEQUENCE CHARACTERISTICS:
      (A) LENGTH: 47 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthesized DNA (cDNA probe)"
   (xi) SEQUENCE DESCRIPTION: SEQ ID NO: 5:
   GGCGAATTCG AAAACCTGTA TTTTCAGGGG CCCTGGGCTA ATATATG 47
(2) INFORMATION FOR SEQ ID NO: 6:
   (i) SEQUENCE CHARACTERISTICS:
      (A) LENGTH: 26 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthesized DNA (cDNA probe)"
   (xi) SEQUENCE DESCRIPTION: SEQ ID NO: 6:
   CGCAAGCTTT TACAGGTATG CAGTAG 26
(2) INFORMATION FOR SEQ ID NO: 7:
   (i) SEQUENCE CHARACTERISTICS:
      (A) LENGTH: 28 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthesized DNA (cDNA probe)"
   (xi) SEQUENCE DESCRIPTION: SEQ ID NO: 7:
   GCGGGATCCC CGGGCCATGG GCTAATAT 28
(2) INFORMATION FOR SEQ ID NO: 8:
   (i) SEQUENCE CHARACTERISTICS:
      (A) LENGTH: 26 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthesized DNA (cDNA probe)"
   (xi) SEQUENCE DESCRIPTION: SEQ ID NO: 8:
   CGCGGATCCT TACAGGTATG CAGTAG                                26
(2) INFORMATION FOR SEQ ID NO: 9:
   (i) SEQUENCE CHARACTERISTICS:
      (A) LENGTH: 98 amino acids
      (B) TYPE: amino acid
      (C) STRANDEDNESS: double
      (D) TOPOLOGY: linear
   (ii) MOLECULE TYPE: protein
   (xi) SEQUENCE DESCRIPTION: SEQ ID NO: 9:
   Gly Pro Trp Ala Ile Ile Cys Ala Gly Lys Ser Ser Asn Glu Ile Arg
   1               5                   10                  15
   Thr Cys Asp Gly His Gly Cys Gly Gln Tyr Thr Ala Gln Arg Asn Gln
               20                  25                  30
   Lys Leu His Gln Gly Val Asp Val Leu Cys Ser Asp Gly Ser Thr Val
           35                  40                  45
   Tyr Ala Pro Phe Thr Gly Ile Met Gly Gln Glu Lys Pro Tyr Lys Asn
       50                  55                  60
   Ile Ser Gly Gly Gly Phe Cys Ile Lys Tyr Lys Gly Ser Ile Val Tyr
   65                  70                  75                  80
   Pro Gly Ile Gln Ser His Ile His Ile Glu Asn Cys Asp Leu Ser Asp
                   85                  90                  95
   Pro Thr

## Description

### TECNICAL FIELD

This invention relates to a method and a kit for measuring the human LECT2.

### BACKGROUND ART

Recently, neutrophilis became known to participate in the damage of cancer cells. As some cancer tissues are observed to have been infiltrated with neutrophilis, it is believed that neutrophilis respond to chemotactic factors secreted from cancer cells.

LECT2 (Leukocyte-derived chemotaxin 2) was discovered during the search for such chemotactic factors secreted from cancer cells, and this seems to be a chemotactic factor which has been obtained from a culture supernatant of T-cell leukemia cell SKW-3.

Human LECT2 was newly discovered as a protein having equal to or more than 90% homology to bovine LECT2 based on human cDNA libraries, using DNA coding LECT2 in bovine serum which is included in a culture supernatant of T-cell leukemia cell SKW-3. From the following Table 1, the amino acid sequence of the human LECT2 can be compared with that of the bovine LECT2.

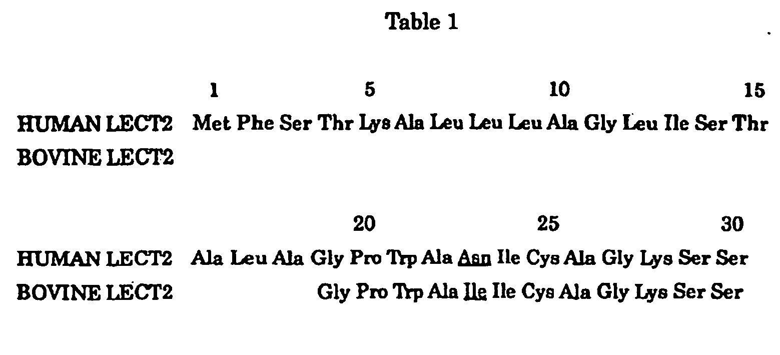

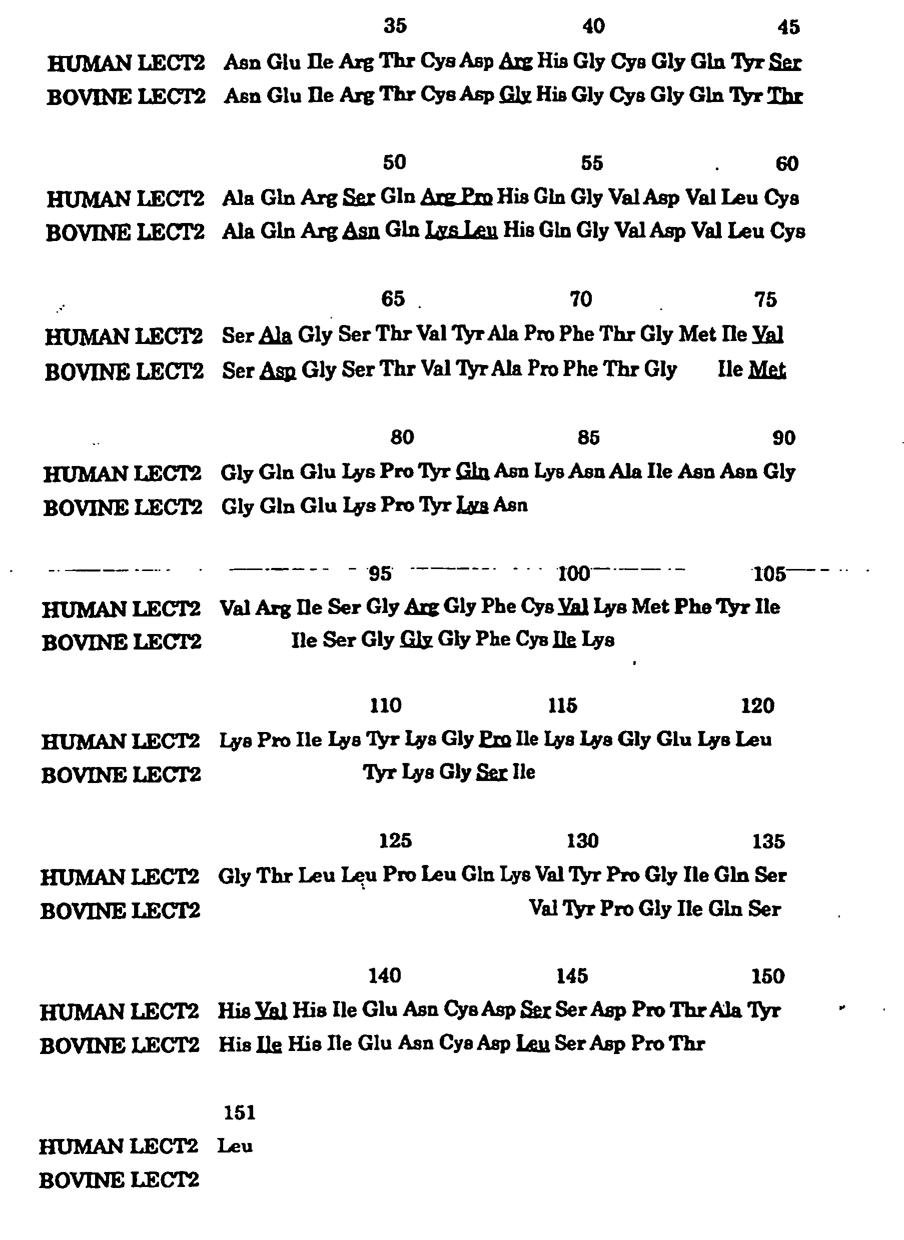

Since the human LECT2 is believed to be a chemotactic factor similar to the bovine LECT2 and its applications to grasping disease conditions and to treatment of cancer are expected, a method for measuring the human LECT2 has been desired to be established.

At first, the bovine LECT2 and the human LECT2 used to be called LECT2a and LECT2b, respectively. However, these names were thought to be inappropriate and therefore changed.

The invention is presented in respect of the aforementioned background, aiming to provide a method and a kit for measuring the human LECT2.

### DISCLOSURE OF THE INVENTION

The invention discloses that specifically react with human LECT2 (a protein having an amino acid sequence indicated in Sequence ID No. 1 in the sequence table). Such antibodies include, for example, an antibody produced by syncytium clone G2A5D7 (Accession No. FERM P-15638), an antibody produced by synsytium clone A1G1C6 (Accession No. FERM P-15639), an antibody produced by syncytium clone 5C5 (Accession No. FREM P-15640), an antibody produced by syncytium clone H12D10D6 (Accession No. FERM P-15641) and an anti-body produced by syncytium clone 89F2 (Accession No. FERM P-16229).

The syncytia disclosed in the invention are characterized by their production of the antibodies which specifically react with the human LECT2. Such syncytia include, for example, syncytium clones G2A5D7 (Accession No. FERM P-15638), A1G1C6 (Accession No. FERM P-15639), 5C5 (Accession No. FERM P-15640), H12D10D6 (Accession No. FERM P-15641) and 89F2 (Accession No. FERM P-16229).

An in vitro measuring method of the human LECT2 according to this invention characteristically includes the following procedures from a) to c) and from d) to f):
a) The human LECT2 as a standard material is reacted with a solidified antibody obtained by combining a first antibody which specifically reacts with the human LECT2 with an insoluble support medium;
b) then, the product is reacted with a labeled antibody obtained by labeling a second antibody which specifically reacts with the human LECT2 with a labeling substance;
c) and a calibration line is prepared by measuring the quantity of the labeling substance in this reaction product; and
d) said solidified antibody is reacted with a sample;
e) then, reacted with said labeled antibody;
f) the quantity of the labeling substance in this reaction product is measured, and the quantity of the human LECT2 contained in the sample is measured from said calibration line.

A kit for in vitro measuring the human LECT2 according to this invention is characterized by comprising a solidified antibody which is obtained by combining an insoluble support medium, with a first antibody which specifically reacts with the human LECT2, and a labeled antibody which is obtained when a labeling substance is labeled to a second antibody which specifically reacts with the human LECT2.

The first antibody is the antibody produced by syncytium clone G2A5D7 (Accession No. FERM P-15638), the antibody produced by syncytium clone A1G1C6 (Accession No. FERM P-15639), the antibody produced by syncytium clone 5C5 (Accession No. FERM P-15640), the antibody produced by syncytium clone H12D10D6 (Accession No. FERM P-15641) or the antibody produced by syncytium clone 89F2 (Accession No. FERM P-16229). The second antibody is any one of said five antibodies, but preferably different from the first one. Also, the labeling substance; for example, may be chosen from a group of peroxidase, biotin, β -D-galactosidase, alkaline phosphatase and microperoxidase.

By means of the method and the kit for measuring human LECT2 according to the present inventions, a chemotactic factor, human LECT2, contained in a sample can be measured. Therefore, the method and kit are used for the diagnosis of liver diseases that are associated with Hepatitis B and C infections.

The method and kit according to this invention can comprise the diagnosis acute and remission stages of liver diseases that are associated with Hepatitis B and C infections.

### BRIEF EXPLANATION OF DRAWING FIGURES

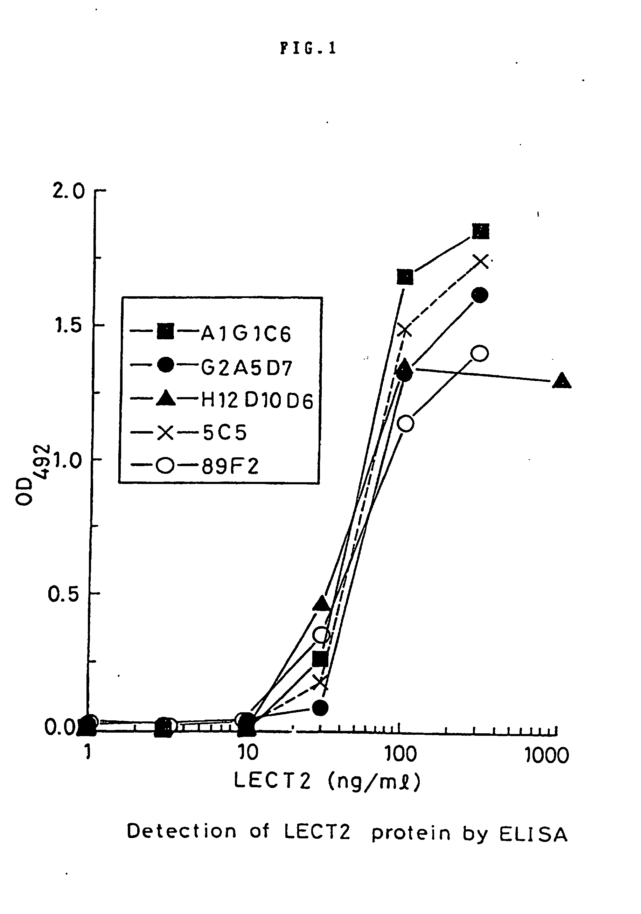

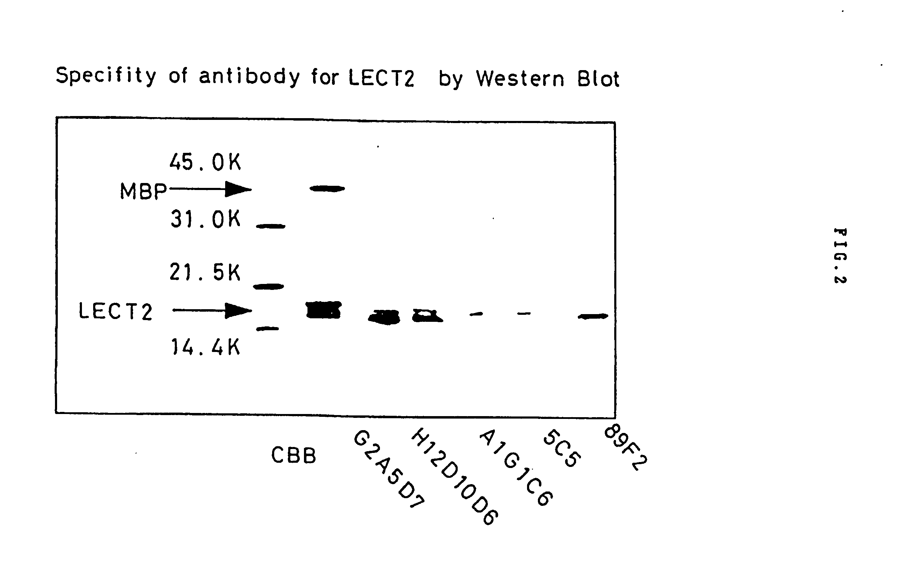

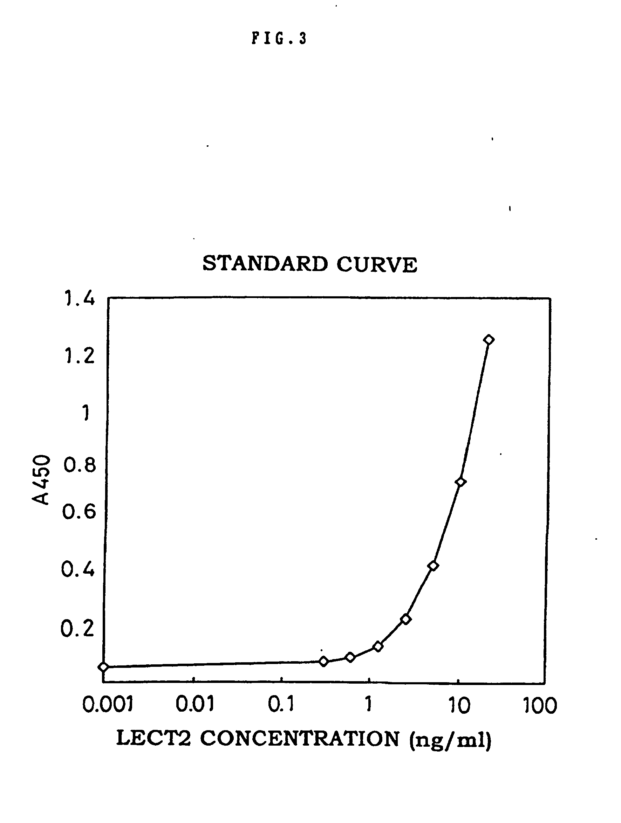

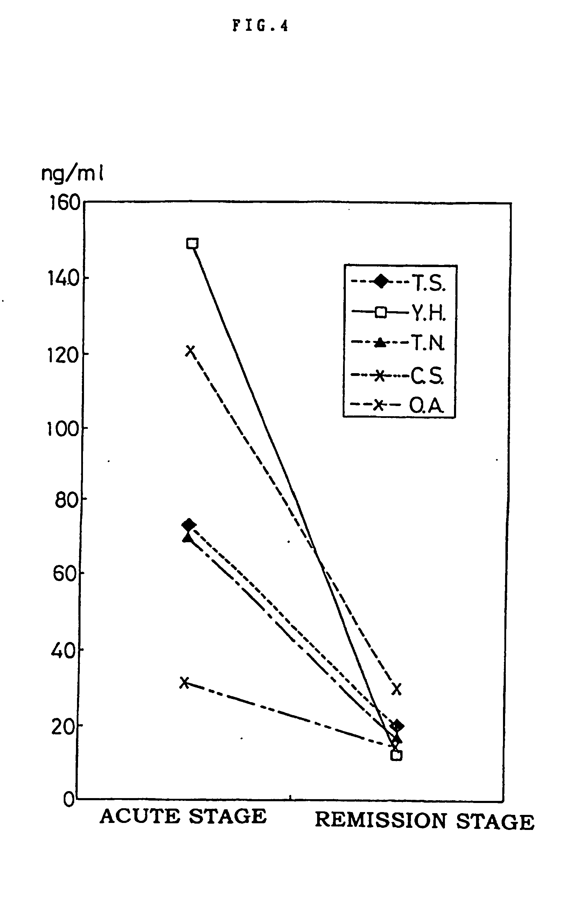

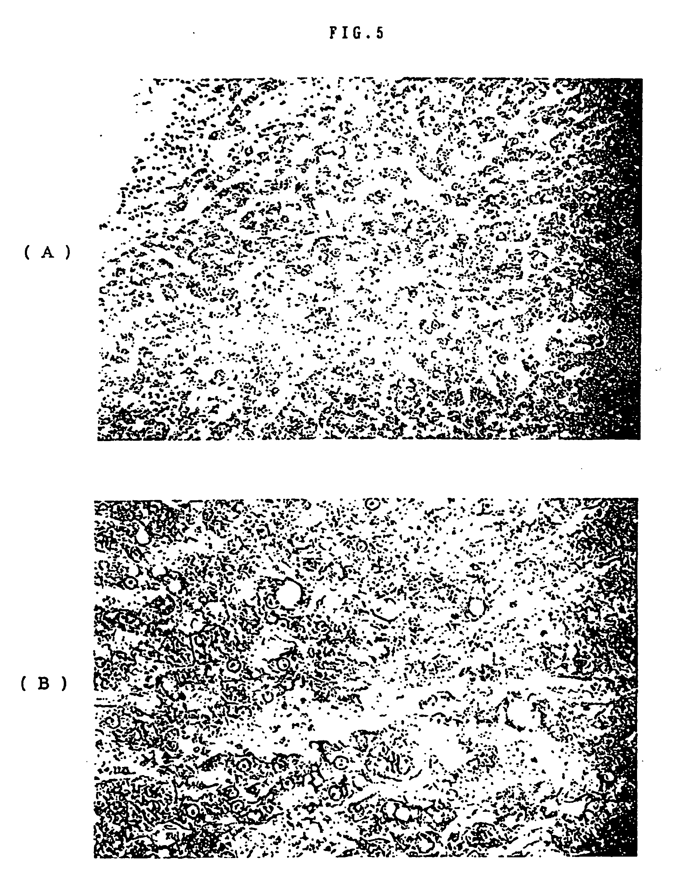

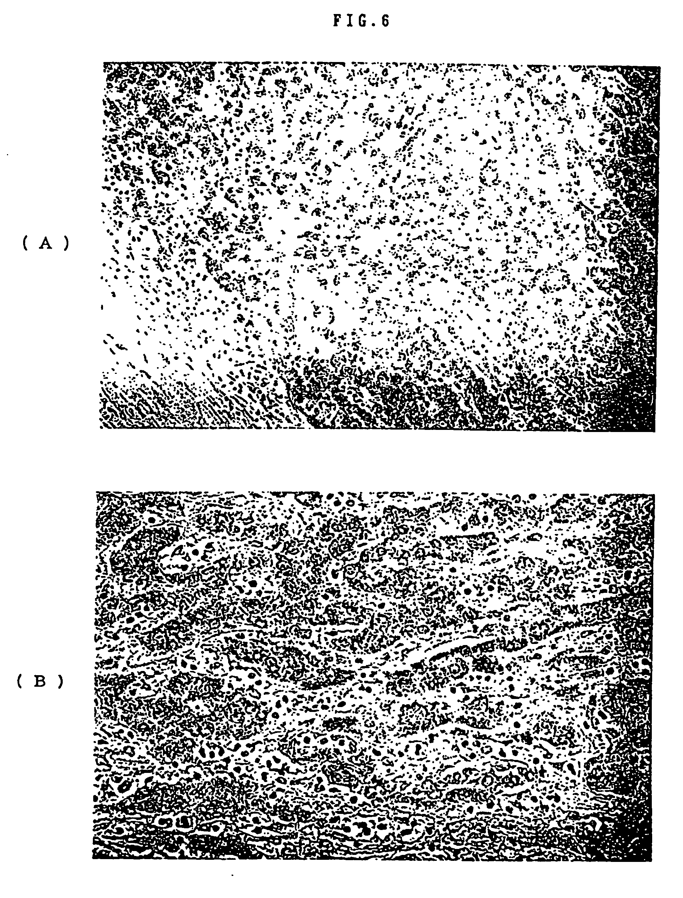

Fig. 1 is a graph showing a detecting result of human LECT2 by ELISA method; Fig. 2 is an exemplary view showing a reaction specificity of a monoclonal antibody of each clone by Western blotting; Fig. 3 is a graph showing a relation between the concentration of human LECT2 and optical absorbance; Fig. 4 is a graph showing measured human LECT2 values of specimens from the patients of acute hepatitis in an acute stage and in a remission stage; Fig. 5 are photographs of dyed tissues of a person in good health, wherein (A) is magnified by 100 times and (B) is magnified by 200 times; and Fig. 6 are photographs of dyed tissues of a patient of cirrhosis, wherein (A) is magnified by 100 times and (B) is magnified by 200 times.

### BEST MODE FOR CARRYING OUT THE INVENTION

### [Antibody which Specifically Reacts with Human LECT2, and Syncytium which Produces the Antibody] - Comparative Example

Procedures for producing an antibody which specifically reacts with the human LECT2 are described as follows.

### A. Antigen

The human LECT2 (a protein having an amino acid sequence in the order shown in Sequence ID No. 1) was cloned and used as an antigen.

### B. Immunity by aforementioned Antigen

As immune animals, mammals such as mice, rats and hamsters can be used. In general, mice are used the most frequently, and BALB/c mice and mice of other strains can be used. In this case, the immune plan and the concentration of an antigen should be chosen so as to produce lymphatic corpuscles which have received enough antigen stimulation. For example, the antigen (25 *µ* g for each time) is injected to the abdominal cavity of a mouse for immunization three times, every once in two weeks, and then to the vein. A few days after the final immunization, cells are taken out from the spleen for fusion.

### C. Cell Fusion

The spleen is taken out in an axenic way from the individual mammal which has thus been immunized. A unicellular suspension is prepared from it. Cell fusion between the spleen cells (cells for producing antibody) and appropriate myeloma cells is carried out using an appropriate cell fusion accelerator. The myeloma cells of a mammal which belongs to the same species as the immunized animal are preferred, however, spleen cells of a rat, a hamster or the like can also be fused with the myeloma cells of a mouse. The preferred ratio of spleen cells to myeloma cells is in the range between ca. 20:1 and ca. 2:1. It is appropriate to use 0.5-1.5ml fusion medium for spleen cells of ca. 10⁸. As a preferred fusion accelerator, for example, polyethylene glycol of mean molecular weight from 1000 to 4000 can be advantageously used. Other fusion accelerators known in this field (Sendai virus (so-called HVJ), for example) can also be used. The cell fusion may also be carried out by using an electric shock method, apart from the method using such fusion accelerators.

### D. Selection of Syncytia which Produce Aimed Antibodies

A mixture of unfused spleen cells, unfused myeloma cells and fused syncytia is diluted in a separate vessel (a microtiterplate, for example) with a selection medium which does not support the unfused myeloma cells, and cultured for a time sufficient to kill the unfused cells (for ca. 1 hour). A drug resistant culture medium (8-azaguanine resistant, for example) which does not support the unfused myeloma cells (HAT culture medium, for example) is used. In this culture medium, the unfused myeloma cells die. The unfused spleen cells are non-neoplastic and therefore die after some definite time (1 week later). Tb the contrary, the fused cells can survive in the selection medium because they have both neoplastic properties of the parent cells of myeloma and properties of parent spleen cells.

Then, after syncytia are detected, antibodies against the aforementioned human LECT2 are screened by Enzyme Linked Immunosorbent Assay, and only syncytia producing monoclonal antibodies which specifically combine with the human LECT2 are selected. Such syncytia include, for example, syncytium clone G2A5D7 (Accession No. FERM P-15638); syncytium clone A1G1C6 (Accession No. FERM P 15639), syncytium clone 5C5 (Accession No. FERM P-15640), syncytium clone H12D10D6 (Accession No. FERM P-15641) and syncytium clone 89F2 (Accession No. FERM P-16229).

### F. Production of Aimed Antibodies

After cloning in an appropriate method (limiting dilution analysis, for example) the syncytia which produce the aimed antibodies, the antibodies can be produced by two different methods. According to the first method, syncytia are cultured in an appropriate culture medium for a predetermined time, and then the monoclonal antibodies produced by the syncytia can be obtained from the culture supernatant. According to the second method, syncytia can be injected to the abdominal cavity of an immune animal having isogenic or semi-isogenic genes. The monoclonal antibodies produced by the syncytia can be obtained from blood and abdominal dropsy of the host animal.

### [Measuring Method of Human LECT2]

### A. Solidified Antibodies

In order to obtain solidified antibodies produced by combining antibodies which specifically react with the human LECT2 with insoluble support mediums, the antibodies and the insoluble support mediums are brought into contact, by which the antibodies are adsorbed on the surfaces of the insoluble support mediums. A chemical procedure such as the covalent bonding is also useful for combining them.

As the insoluble support mediums, for example, high polymers such as polystyrene, polyethylene, polypropyrene, polyester, polyacrylonitrile, fluorine polymer, cross-linked dextran and polysuccaride can be shown, as well as paper, glass, metal, agarose and combinations of these materials. As the form of the insoluble support mediums, various forms can be adapted like a tray, sphere, rod, fiber, plate, vessel, cell, test tube and so on.

### B. Labeled Antibodies

The antibody which specifically reacts with the human LECT2 may be either IgG or a fragment of Fab, F(ab)'₂ or the like.

The labeling substance is not limited if it can be used for ordinary immunological measuring methods, however, enzymes, avidins, fluorescent substances, luminescent substances, radioactive substances and the like are preferably used. As enzymes, peroxidase, β-D-galactosidase, alkaline phosphatase, microperoxidase can be used, as fluorescent substances, fluorescent isothiocyanate, phicobiliprotein, phicoerythrine and the like can be used, as luminescent substances, isolucinol, lucigenine and the like can be used and as radioactive substances, ¹²⁵I, ¹³¹I, ¹⁴C, ³H and the like can be used. When biotine is used as a labeling substance further with avidin labeled with an enzyme, a high sensitivity can be preferably obtained. In this case, an enzyme similar to that labeled to the antibody can be used as a labeling enzyme, peroxidase being especially preferable.

When an enzyme is used as a labeling substance, a substrate and, if needed, a coloring substance are used, in order to measure the activity thereof. When peroxidase is used as an enzyme, H₂O₂ is used as a substrate, and ammonium salt of 2, 2'-azino-di-[3-ethylbenzylthiazoline sulphonic acid](ABTS), 5-aminosalicylic acid, o-phenylenediamine (OPD), 4-aminoantipyrine, 3, 3', 5,5'-tetramethylbenzidine or the like can be used as a coloring substance. When alkaline phosphatase is used as an enzyme, o-nitrophenylphosphate or the like is used as a substrate. When β-D-galactosidase is used as an enzyme, fluorescent di-(β-D-galactopyranose), 4-methylunberypheryl- β-D-galactopyranose or the like can be used as a substrate.

### C. Preparation of Calibration Curve

The human LECT2, as a standard substance, is reacted with a solidified antibody, and then the human LECT2 which has specifically reacted with the solidified antibody is reacted with a labeled antibody. Subsequently, the quantity of the labeling substance of the labeled antibody is measured. By this procedure, a relation between the quantity of the human LECT2 and that of the labeling substance, or a calibration curve is obtained.

As reaction media used in this immunological measuring method, the liquids of pH 6.0 to 8.0, including, for example, buffer solutions of phosphoric acid, of tris-hydrochrolic acid and of acetic acid and the like are shown.

### D. Measurement of Human LECT2 included in samples.

A sample reacted with the solidified antibody, and then reacted with the labeled antibody. In this stage, the labeled antibody reacts depending on the concentration of the human LECT2 included in the sample Subsequently, the quantity of the labeling substance in the labeled antibody is measured. The measured value is referred to the calibration curve, and as the result, the concentration of the human LECT2 in the sample is measured. Concerning the reaction medium, explanation was already given in the aforementioned paragraph C. [Kit for Measuring Human LECT2]

The kit for measuring the human LECT2 comprises at least:
(1) a solidified antibody obtained by combining a first antibody which specifically reacts with the human LECT2 with an insoluble support medium; and
(2) a labeled antibody obtained by labeling a second antibody which specifically reacts with the human LECT2 with a labeling substance. The kit may includes other reaction medium and/or a standard substance (human LECT2). Moreover, when the labeling substance of the labeled antibody is an enzyme , a reaction-terminating solution and a substrate for measuring the activity of the enzyme are usually included. Explanations of the insoluble support medium and the labeling substance were already given before and, therefore, are omitted here. The aforementioned measuring method can be carried out using this measuring kit.

Preferred embodiments of the invention will now be described with reference to drawing figures. However, the embodiment of this invention is not restricted to the following embodiments, and can be variously modified within the scope of the invention.

### [Embodiment 1] Preparation of Immunogen (Human LECT2) - Comparative Embodiment [1-1] Cloning of Human LECT2 and Determination of Nucleotide

### Sequence of Cloned Human LECT2

The cloning of the human LECT2 cDNA was carried out as follows.

Poly A* RNA was prepared by treating the T cell-type leukemia cell SKW-3 with PHA-P (50 *µ* g/ml) (made by DIFCO Inc.). The first strand cDNA (1st cDNA) was synthesized using poly A⁺ RNA (5 *µ* g) and oligo-dT as a primer.

Then, a primer of PCR was synthesized based on the partial amino acid sequence of bovine LECT2 (its amino acid sequence is shown in Sequence ID No. 9). Namely, 6 kinds of oligonucleotides were deduced from the amino acid sequence WAIICA to form 5' primer, and 4 kinds of oligonucleotides were deduced from the amino acid sequence HIENCD to form 3' primer. The DNA fragment was amplified by the 24 reactions which am the combinations of said primers using the 1st cDNA as a template according to PCR method. The amplified DNA was separated with agarose gel using DNA probe (GATGTC/GCTA/GTGCTCT/CGATGGC/GTCT/CACT/AGTC/GTATGCT/CC CT/CTT: Refer to Sequence ID No. 4), based on amino acid sequence DVLCSDGSTVYKPF, and analyzed by Southern blotting method. As the result, ca. 370bp of DNA fragments were detected, which were cloned to pUC19.

As the result of screening cDNA library of a human liver using the cloned cDNA fragment as a probe, twelve positive clones were obtained among 1.3 million clones. All these clones proved to be classified into two types by the analysis of restriction enzymes, and the base sequence of the clone of the longest cDNA fragment of each group was determined. The sequence analysis of the longest clones of each type suggested that the two types of cDNA would be derived from an identical gene which had two poly A signals on 3' side. The amino acid sequence of the coded protein revealed that the amino acid sequences were 90% homologous to the determined bovine LECT2 amino acid sequence. Then, this protein, considered to be coded, was named human LECT2. The amino acid sequence and the base sequence of the human LECT2 are shown in Sequence ID NOs. 1 and 2, respectively.

### [1-2] Constitution of Recombinant Plasmid containing Human LECT2 Gene

Based on the cloned human LECT2 CDNA, GGCGAATTCGAAAACCTGTATTTTCAGGGGCCCTGGGCTAATATATG (Refer to Sequence ID No. 5) was used as the 5'-primer and CGCAAGCTTTTACAGGTATGCAGTAG (Refer to Sequence ID No. 6) was used as the 3'-primer. The DNA fragments including the human LECT2 cDNA were amplified with these primers by PCR method having 25 cycles of denaturation at 94°C for 1 min., annealing at 55°C for 2 min. and elongation at 72°C for 3 min. After digested with EcoRI and HindIII, the fragments including the human LECT2 cDNA were ligated into the EcoRi/HindIII site of pMAL™-C (Biolab Inc.). Thus recombined- plasmid was named pMAL-TEV-human LECT2. This manifestation vector can produce a fused protein of the maltose-binding-protein and the human LECT2 in the presence of IPTG under the control of tac promoter. A fragment of the TEV protease (originated from the Tabacco Etch Virus) exists in its coupling site, which enables a specific fragmentation.

Also, based on the cloned human LECT2 cDNA, GCGGGATCCCCGGGCCATGGGCTAATAT (Refer to Sequence ID No. 7) was used as the 5'-primer and CGCGGATCCTTACAGGTATGCAGTAG (Refer to Sequence ID No. 8) was used as the 3'-primer. The DNA fragments including the human LECT2 cDNA were amplified with these primers by PCR method having 25 cycles of denaturation at 94°C for 1 min., annealing at 55°C for 2 min. and elongation at 72°C for 3 min. After digested with BamHI, the fragments including the human LECT2 cDNA were ligated into the BamHI site of pGEX-3X (Pharmacia Inc.). Thus recombined plasmid was named pGEX-Xa-human LECT2. This manifestation vector can produce a fused protein of a glutathione-S-transphelase and the human LECT2 in the presence of IPTG under the control of tac promoter. A fragment of the Xa protease exists in its coupling site, which enables a specific fragmentation.

### [1-3] Transformation of E. Coli by Recombinant Plasmid containing Human LECT2 Gene

E. coli JM109 was transformed with the recombinant plasmid pMAL-TEV-human LECT2 obtained above. Out of thus obtained E. coli clones, those having an expected base sequence were screened by the deoxy method and E. coli clone Mal-human LECT2 (Accession No. FERM P. 14669: It was transferred to the international deposit under Accession No. FERM BP-5302), having an expected DNA fragment, was obtained.

### [1-4] Production of Human LECT2 by Animal Cells

The 5' side of the human LECT2 cDNA ligated into the BamHI site of pUC19 from HindIII to EcoRI was deleted by exonuclease m until -14 base sequence, treated with T4 DNA polymerase to form a smooth end group, ligated with Pstl linkers, fragmented with Pstl and BglII and ligated into the Pstl/BamHI site of the manifestation vector pcDL-SR α 294. This recombinant manifestation vector was transfected into CHO cells of a Chinese hamster, and one strain which highly manifests the human LECT2 (C1D8-1) (Accession No. FERM P-14668: It was transferred to the international deposit under Accession No. FERM BP-5301) was obtained.

### (Determination of Molecular Size)

The recombinant human LECT2 (expression of animal cells) was defected by metabolically labeling it ³⁵S-methionine and subjecting the culture supernatant of CHO cells to SDS gel electrophoresis, giving two bands of molecular size of ca. 14kDa and 16kDa. (The 16kDa band is main. The two bands seem to have appeared because of differences in processing.)

### [Embodiment 2] Immunity - Comparative Embodiments

The immunogen, human LECT2, prepared in the aforementioned

### Embodiment 1, (100 µl) was mixed sufficiently with Freund's complete

adjuvant (100 µl) to give a suspension, which was injected to the abdominal cavities of two mice (male BALB/c), 25 µl to each as the immunogen. Further, the immunogen of the same quantity was injected 5 times, every other week. Then, three days later the spleens were taken out and subjected to a fusion experiment.

Separately, two rabbits were also immuned in the same manner. After serum was separated from the blood taken out from the rabbits, absorption operation was carried out using an ordinary method, and the IgG fraction was separated to produce a polyclonal antibody.

### [Embodiment3] Cell Fusion and Selection and Acquisition of Syncytia which Produce Aimed Monoclonal Antibodies - Comparative Embodiment

The spleen cells taken out from the mice (10 parts) and myeloma cells (SP-2/O-Ag-14) from mice of the same strain (1part) were mixed, and cell fusion was carried out using 50% polyethyrene glycol 4000 as a fusion accelerator. The fused cells were suspended in the HAT culture medium (culture medium containing hypoxanthine, amino pterin and thymidine) containing 10% bovine serum so as to give the cell concentration of 1 × 10⁶ cells/ml, and poured to 96 wells of microtiterplate (Maxisoap made by Nunk Inc.: Same ones were used hereafter), by 100µl for each well.

The syncytia were cultured in CO₂ incubator (5% CO₂, 37 °C), transferred to the HAT culture medium, conditioned in the HAT culture medium, and further conditioned in the 10% FCS-RPMI 1640 culture medium.

The antibodies in the supernatant of the fused and cultured cells were detected, using a microtiterplate on which the human LECT2 protein was solidified, by ELISA method. Cloning was carried out two times for every well which proved to be positive, using the limiting dilution analysis. Then, 5 kinds of clones which had reactivity to the human LECT2 were selected, and named G2A5D7, AlGlC6, 5C5, H12D10D6 and 89F2, respectively (Accession Nos. FERM P.15638. FERM P.15639. FERM P-15640, FERM P. 15641 and FERM P-16229, respectively).

Each clone obtained was suspended in the 90% bovine serum including 10% DMSO and kept in the liquidified nitrogen. The monoclonal antibody produced by each clone was amplified in the abdominal cavity of a mouse. Each antibody from the abdominal dropsy was purified with the protein-A cephalose column.

### [Embodiment 4] Confirmation of Specificity of Monoclonal Antibodies - Comparative Embodiment

### 1. Confirmation of Specificity by ELISA Method

A PBS solution of the human LECT2 (1-1000ng/ml) was prepared. The human LECT2 was solidified on a microtiterplate using the solution, reacted with the antibody solution of each clone, and then reacted with the anti-mouse immunoglobulin labeled with peroxidase (made by Cappel Inc.). Optical absorbance at the wave length of 492nm was measured for each product in a solution of orthophenylene diamine and hydrogene peroxide as a substrate. The results are shown in Fig. 1.

It was confirmed that each clone specifically reacts with the human LECT2 from the fact that the value of optical absorbance increased with the concentration of the solidified human LECT2 and became constant above some concentration.

### 2. Confirmation of Specificity by Western Blotting

A mixture of the human LECT2 and the maltose-binding-protein (MBP), as an antigen, was subjected to SDS-polyacrylamide gel electrophoresis (PAGE) (16.5% mono-acrylamidelbis-acrylamide; 3% bis/mono-acrylamide + bis). After the electrophoresis, a specificity of the reaction was confirmed by Western blotting. The monoclonal antibody of each clone showed reactivity at about 16kD, and did not react with the MBP. The results are shown in Fig. 2.

In Fig. 2, CBB is the abbreviation of cummarcy brilliant blue and shows protein dyeing. The first lane is a molecular size marker and the others are electrophoresis results of the human LECT2 + MBP, as an antigen. The first and second lanes show the protein dyeing and the others show Western blot for each monoclonal antibody.

### [Embodiment 5] Confirmation of Subclass by ELISA Method Comparative Embodiment

For each monoclonal antibody obtained in the aforementioned Embodiment 3, the class- subclass was confirmed using a monoclonal subclass isotyping kit made by American Corlex Inc. The result was that clones AlGlC6 and G2A5 were IgG2b and clone H12D10 was IgM. However, clones 5C5 and 89F2 were not confirmed.

### [Embodiment 6] Solidification of Antibodies - Comparative Embodiment

The solution of monoclonal antibody clone G2A5, obtained in the aforementioned Embodiment 3, was prepared at the concentration of 5 µl/ml, using 0.1M carbonate buffer (pH 9.0), poured on 96 wells of microtiterplate by 100 µl for each well, and reacted stationarily at 4°C for 20 hours. The antibody solution was eliminated from the wells, 200 µl of PBS containing 1% BSA and 5% succarose was added to each well and blocking was carried out at room temperature (20-25°C) for 2 hours in the stationary state. The blocking liquid was discarded and the plate was air-dried to give solidified antibodies. The solidified antibodies were kept in a sealed eondition with desiccant.

### [Embodiment 7] Preparation of Labeled Antibodies Comparative Embodiment

Purified IgG of the polyclonal antibody, obtained in the aforementioned Embodiment 2, was added with ficin by 0.056U for 1mg purified IgG, reacted at 37°C for 8 hours, and subjected to gel filtration using Ultrogel ACA44, and Fab' fraction was thus obtained. This Fab' fraction was labeled with peroxidase by the maleimide method to give peroxidase-labeled antibodies. Specifically, the labeling was carried out following "Measuring Method of Enzyme Immunity, the Third Edition," published by Igakushoin and written by Eiji Ishikawa.

### [Embodiment 8] Measurement of Human LECT2

The human LECT2 protein was diluted with PBS to give 0.001-20ng/ml solutions. 200 µl of each solution was added to each well of the plate with the solidified antibodies obtained in the aforementioned Embodiment 6, and reacted at room temperature for 1 hour. After each well was washed with 300 µl of PBS 4 times, excessive PBS was removed. Then, each well was added with the peroxidase-labeled antibodies (100 µl) obtained in the aforementioned Embodiment 7, reacted at room temperature for 1 hour, washed again with PBS, added and reacted with a solution of tetramethyl benzidine and hydrogen peroxide (100 µl), and the reaction was terminated by adding 100 µl of 1.5N phosphoric acid. Then, the optical absorbance at the wave length of 450nm was measured. The results of measurement are shown in Table 2 and Fig. 3.

**Table 2**

| Std. conc. (ng/ml) | A450 |
|---|---|
| 0.001 | 0.05 |
| 0.3 | 0.07 |
| 0.6 | 0.085 |
| 1.25 | 0.125 |
| 2.5 | 0.22 |
| 5 | 0.41 |
| 10 | 0.73 |
| 20 | 1.25 |

From the results, the relation of the quantity of the labeled substance to that of the human LECT2 is obtained, that is, a calibration curve is prepared. By using the calibration curve, the relation of the quantity of the labeled substance to that of the human LECT2 in a sample is obtained, that is, a calibration curve is prepared, which is used to know the quantity of the human LECT2 contained in the specimen.

For samples from acute hepatitis patients, data of a comparison of measured values at acute stages and at remission stages are shown in Table 3 and Fig. 4.

**Table 3**

| Specimen | Disease | Disease Condition | Measured value (ng/ml) |
|---|---|---|---|
| T.S. | HA (B-type) | Acute Stage | 73 |
| | | Remission Stage | 21 |
| Y.H. | HA (B-type) | Acute Stage | 149 |
| | | Remission Stage | 13 |
| T.N. | HA (B-type) | Acute Stage | 70. |
| | | Remission Stage | 18 |
| C.S. | HA (C-type) | Acute Stage | 32 |
| | | Remission Stage | 15 |
| O.A. | HA (resistant) | Acute Stage | 121 |
| | | Remission Stage | 31 |

As the result of a preliminary study on the measured series, it was proved that the human LECT2 in samples lost its antigenicity at a comparative early stage, especially, in serum the antigencity disappeared promptly. Therefore, plasma was used as a sample. Plasma was obtained by quickly separating the blood after the blood collection and stored under the frozen state at -20°C or lower until measurement. When the sample is not diluted by more than 5 times, there is a possibility that measured values are influenced. Therefore, the sample was measured after diluted by 10 times.

Comparison of the measured values of each sample at its acute stage and at its remission stage shows distinct decline of the data of the remission stage compared to that of the acute stage. From this result, the concentration of the human LECT2 in samples is thought to reflect the disease conditions of patients.

As, the content of the human LECT2, a chemotactic factor, in a sample can be measured in this way, the result of measurement can be used to grasp disease conditions and to cure the disease.

### [Embodiment 9] Dyeing of Immunity Tissue

Liver tissues were taken out from a person in good health and a hepatitis patient, subjected to formalin 5xation using an ordinary method, and embedded with parafin. The parafin-embedded tissues were cut with a microtome into tissue sections. Deparafinization of the tissue sections was carried out by an ordinary method. About 100 *µ*l of PBS solution of IgG traction (5 *µ* g/ml), obtained by purifying monoclonal antibody clone 89F2 in Embodiment 3, was poured on the tissue sections, reacted at 37°C for 30 minutes and the tissue sections were washed with PBS. The sections were then reacted with the anti-mouse IgG labeled with peroxidase (made by Daco Inc.) at 37 °C for 30 minutes, washed again with PBS and, subsequently, dyed by reacting with a solution of 3, 3', 5, 5'-diamino benzidine and hydrogen peroxide. The nuclei were dyed with hematoxylin.

Fig. 5 are photographs showing the dyed images of the tissues taken out from a person in good health; (A) is a photograph magnified by 100 times and (B) is a photograph magnified by 200 times. Fig. 6 are photographs showing the dyed images of the tissues taken out from a hepatitis patient; (A) is a photograph magnified by 100 times and (B) is a photograph magnified by 200 times.

Diffuse dyeing was observed on the cytoplasm of liver cells for both tissue sections from a person in good health and a hepatitis patient. Specifically, for both of the person in good health and the hepatitis patient, granular dyeing (dyed brown) by diaminobenzidine was observed on parenchyma cells of livers, but for the hepatitis patient, mixtures of positive and negative cells were confirmed and the dyeing pattern was different from that of the person in good health (for the person in good health, parenchyma cells of the liver were all positive). Dyeing of connective tissues was not observed. The particles which are dyed blue like spots in the photographs of Figs. 5 and 6 are nuclei. The fact suggests that the LECT2 has some relation with the cell cycle.

### POSSIBILITY OF INDUSTRIAL USE

The antibody against the human LECT2 described in this invention is useful to the immunological medical treatment and diagnosis, and useful in the industrial fields related thereto. The syncytium described in this invention is also useful for obtaining the antibody against the human LECT2. Furthermore, the antibody against the human LECT2 described in this invention can be used for a method and a kit for measuring the human LECT2. As the human LECT2 (thought to be a chemotactic factor) contained in a sample can be measured using the method and the kit for measurement, it becomes effectively possible to use and apply the result of measurement for diagnosing liver diseases that are associated with Hepatitis B and C inflections. In more detail, for example, the antibody described in this invention reacted with the tissues taken out from the patients of various diseases, (hepatitis and cirrhosis, for example) and the sites in the tissues where the cells manifesting the human LECT2 are located can be examined. Accordingly, the method and the kit of the invention can be extended to the diagnosis of liver diseases that are associated with Hepatitis B and C infections.

### DEPOSITARY INSTITUTION

C1D8-1 and Mal-human LECT2 were deposited with the following international depositary institution, under the following accession numbers and deposit dates, respectively.

| | |
|---|---|
| Name of institution: | National Institute of Bioscience and Human-Technology, Agency of Industrial Science and Technology, Ministry of International Trade and Industry |
| Address: | 1-3, Higashi 1-chome, Tsukuba city, Ibaraki pref., Japan (Zip Cord 305) |

Accession Number and Deposit Date:
(1) C1D8-1
   FERM BP-5301: Nov. 25,1994
(2) Mal-human LECT2
   FERM BP 5302: Nov. 25,1994

Syncytium clones, G2A5D7, A1G1C6, 5C5, H12D10D6 and 89F2 were deposited with the following domestic depositary institution, under the following accession numbers and deposit dates, respectively.

| | |
|---|---|
| Name of institution: | National Institute of Bioscience and Human-Technology, Agency of Industrial Science and Technology, Ministry of International Trade and Industry |
| Address: . | 1-3, Higashi 1-chome, Tsukuba city, Ibaraki pref., Japan (Zip Cord 305) |

Accession Number and Deposit Date:
(1) Syncytium clone G2A5D7
   FERM P-15638: May 21,1996
(2) Syncytium clone A1G1C6
   FERM P-15639: May 21,1996
(3) Syncytium clone 5C5
   FERM P-15640: May 21,1996
(4) Syncytium clone H12D10D6
   FERM P-15641: May 21,1996
(5) Syncytium clone 89F2
   FERM P-16229: May 19,1997

### [Sequence Table]

Sequence ID No.: 1
Length of sequence: 151
Type of sequence: amino acid
Topology: linear chain
Molecular type of sequence: Protein
Specificity of sequence:
   Position: 58
   Other Information: Xaa = Val or Ile
Sequence description
Sequence ID No.: 2
Length of sequence: 1092
Type of sequence: nucleic acid
Strandness: 2
Topology: linear chain
Molecular type of sequence: cDNA
Original source: human
Cell type: liver
Specificity of sequence:
   Mark describing specificity: 5'UTR
   Location: 1..200
   Method for determination of specificity: P

   Mark describing specificity: 3'UTR
   Location: 657..1092
   Method for determination of specificity: P

   Mark describing specificity: CDS
   Location: 201..656
   Method for determination of specificiy: P

   Mark describing specificity: mutation
   Location: replace (372, "a")
      replace (748, "g")
      replace (961, "c")
      replace (967, "c")
   Method for determination of specificity: E
   Mark describing specificity: polyA signal
   Location: 684..689
      1060..1065
   Method for determination of specificity: E
Sequence description
Sequence ID No.: 3
   Length of sequence: 1092
   Type of sequence: nucleic acid
   Strandness: 2
   Topology: linear chain
   Molecular type of sequence: cDNA
   Original source: human
   Cell type: liver
   Specificity of sequence:
      Mark describing specificity: 5'UTR
      Location: 1..200
      Method for determination of specificity: P

      Mark describing specificity: 3'UTR
      Location: 657..1092
      Method for determination of specificity: P

      Mark describing specificity: CDS
      Location: 201..656
      Method for determination of specificity: P

      Mark describing specificity: mutation
      Location: replace (372, "a")
         replace (748, "g")
         replace (961, "c")
         replace (967, "c")
      Method for determination of specificity: E
      Mark describing specificity: polyA signal
      Location: 684..689
         1060..1065
      Method for determination of specificity: E
Sequence description
Sequence ID No.: 4
Length of sequence: 41
Type of sequence: nucleic acid
Topology: linear chain
Molecular type of sequence: other nucleic acid
   synthesized DNA (cDNA probe)
Specificity of sequence:
   Location: 6
      Other information: N = C or G
   Location: 9
      Other information: N = A or G
   Location: 15
      Other information: N = T or C
   Location: 21
      Other information: N = C or G
   Location: 24
      Other information: N = T or C
   Location: 27
      Other information: N = T or A
   Location: 30
      Other information: N = C or G
   Location: 36
      Other information: N = T or C
   Location: 39
      Other information: N = T or C
Sequence description
Sequence ID No.: 5
Length of sequence: 47
Type of sequence: nucleic acid
Topology: linear chain
Molecular type of sequence: other nucleic acid
   synthesized DNA (primer)
Sequence description
Sequence ID No.: 6
Length of sequence: 26
Type of sequence: nucleic acid
Topology: linear chain
Molecular type of sequence: other nucleic acid
   synthesized DNA (primer)
Sequence description
Sequence ID No.: 7
Length of sequence: 28
Type of sequence: nucleic acid
Topology: linear chain
Molecular type of sequence: other nucleic acid
   synthesized DNA (primer)
Sequence description
Sequence ID No.: 8
Length of sequence: 26
Type of sequence: nucleic acid
Topology: linear chain
Molecular type of sequence: other nucleic acid
   synthesized DNA (primer)
Sequence description
Sequence ID No.: 9
Length of sequence: 98
Type of sequence: amino acid
Topology: linear chain
Molecular type of sequence: protein
Sequence description

## Claims

1. An in vitro measuring method of human LECT2 for the diagnosis of liver diseases that are associated with Hepatitis B and C infections, **characterized by** including the following procedures from a) to c) and from d) to f):
a) the human LECT2 as a standard material is reacted with a solidified antibody obtained by combining a first antibody which specifically reacts with the human LECT2 with an insoluble support medium;
b) then, the product is reacted with a labeled antibody obtained by labeling a second antibody which specifically reacts with the human LECT2 with a labeling substance;
c) and a calibration line is prepared by measuring the quantity of the labeling substance in the reaction product; and
d) said solidified antibody is reacted with a sample;
e) then, reacted with said labeled antibody;
f) and the quantity of the labeling substance in the reaction product is measured and the quantity of the human LECT2 contained in the sample is measured from said calibration line.

2. A kit for in vitro measuring of human LECT2 for the diagnosis of liver diseases that are associated with Hepatitis B and C infections, **characterized by** comprising:
a solidified antibody obtained by combining a first antibody which specifically reacts with the human LECT2 with an insoluble support medium;
a labeled antibody obtained by labeling a second antibody which specifically reacts with the human LECT2 with a labeling substance.

3. The method of claim 1 comprising the diagnosis at acute and remission stages of liver diseases that are associated with Hepatitis B and C infections.

4. The kit of claim 2 comprising the diagnosis at acute and remission stages of liver diseases that are associated with Hepatitis B and C infections.

## Patentansprüche

1. In vitro Messverfahren von menschlichem LECT2 für die Diagnose von Lebererkrankungen, die mit Hepatitis B und C Infektionen assoziiert sind, **gekennzeichnet durch** das Einschließen der folgenden Verfahrensschritte von a) bis c) und von d) bis f):
a) das menschliche LECT2 als ein Standardmaterial wird mit einem befestigten Antikörper reagiert, der **durch** Kombinieren eines ersten Antikörpers, der spezifisch mit dem menschlichen LECT2 reagiert, mit einem unlöslichen Trägermedium erhalten wurde;
b) dann wird das Produkt mit einem markierten Antikörper reagiert, der **durch** Markieren eines zweiten Antikörpers, der spezifisch mit dem menschlichen LECT2 reagiert, mit einer Markierungssubstanz erhalten wurde;
c) und eine Eichlinie wird **durch** Messen der Menge der Markierungssubstanz in dem Reaktionsprodukt erzeugt; und
d) der befestigte Antikörper wird mit einer Probe reagiert;
e) dann mit dem markierten Antikörper reagiert;
f) und die Menge der Markierungssubstanz in dem Reaktionsprodukt wird gemessen und die Menge des menschlichen LECT2, die in der Probe enthalten ist, wird von der Eichlinie gemessen.

2. Kit für die in vitro Messung von menschlichem LECT2 für die Diagnose von Lebererkrankungen, die mit Hepatitis B und C Infektionen assoziiert sind, **gekennzeichnet durch** Umfassen von:
einem befestigten Antikörper, der **durch** Kombinieren eines ersten Antikörpers, der spezifisch mit dem menschlichen LECT2 reagiert, mit einem unlöslichen Trägermedium erhalten wurde;
einem markierten Antikörper, der **durch** Markieren eines zweiten Antikörpers, der spezifisch mit dem menschlichen LECT2 reagiert, mit einer Markierungssubstanz erhalten wurde.

3. Verfahren nach Anspruch 1, umfassend die Diagnose bei akuten und Remissionsphasen der Lebererkrankungen die mit Hepatitis B und C Infektionen assoziiert sind.

4. Kit nach Anspruch 2, umfassend die Diagnose bei akuten und Remissionsphasen der Lebererkrankungen die mit Hepatitis B und C Infektionen assoziiert sind.

## Revendications

1. Procédé de mesure *in vitro* des LECT2 humains pour le diagnostic de maladies hépatiques qui sont associées aux infections hépatites B et C, **caractérisé en ce qu'**il comprend les procédures suivantes a) à c) et d) à f) :
a) on fait réagir les LECT2 humains en tant qu'un matériau étalon avec un anticorps solidifié obtenu par la combinaison d'un premier anticorps qui réagit spécifiquement avec les LECT2 humains et d'un milieu de support insoluble ;
b) on fait alors réagir le produit avec un anticorps marqué obtenu par le marquage d'un second anticorps qui réagit spécifiquement avec les LECT2 humains avec une substance de marquage ;
c) et on prépare une courbe d'étalonnage en mesurant la quantité de la substance de marquage dans le produit de réaction ; et
d) on fait réagir ledit anticorps solidifié avec un échantillon ;
e) on le fait ensuite réagir avec ledit anticorps marqué ;
f) puis on mesure la quantité de la substance de marquage dans le produit de réaction et on mesure la quantité de LECT2 humains contenus dans l'échantillon en se basant sur ladite courbe d'étalonnage.

2. Kit de mesure *in vitro* des LECT2 humains pour le diagnostic de maladies hépatiques qui sont associées aux infections hépatites B et C, **caractérisé en ce qu'**il comprend :
un anticorps solidifié obtenu par combinaison d'un premier anticorps qui réagit spécifiquement avec les LECT2 humains et d'un milieu de support insoluble ;
un anticorps marqué obtenu par marquage d'un second anticorps qui réagit spécifiquement avec les LECT2 humains avec une substance de marquage.

3. Procédé selon la revendication 1, comprenant le diagnostic à des stades aigu et de rémission de maladies hépatiques qui sont associées aux infections hépatites B et C.

4. Kit selon la revendication 2, comprenant le diagnostic à des stades aigu et de rémission de maladies hépatiques qui sont associées aux infections hépatites B et C.
